# EUROPEAN PATENT SPECIFICATION

(11) **EP 2 994 144 B1**
(45) Date of publication and mention of the grant of the patent: **23.11.2016**
(21) Application number: 14728084.6
(22) Date of filing: 07.05.2014
(51) Int. Cl.: A61K 31/6615, A61K 31/70, A61K 31/7016, A61K 31/716, A61K 31/728, A61P 17/00, A61P 15/02, A61P 13/02

(54) **TOPICAL PREPARATIONS COMPRISING HYALURONIC ACID, VERBASCOSIDE AND GLYCEROPHOSPHOINOSITOL**
TOPISCHE ZUSAMMENSETZUNGEN, WELCHE HYALURONSÄURE, VERBASCOSIDE UND GLYCEROPHOSPHOINOSITOL ENTHALTEN
PREPARATIONS TOPIQUES COMPRENANT ACID HYALURONIQUE, VERBASCOSIDE ET GLYCEROPHOSPHOINOSITOL

(30) Priority: 09.05.2013 IT MI20130763
(43) Date of publication of application: 16.03.2016
(73) Proprietor: ALTERGON S.A., CH-6900 Lugano (CH)
(72) Inventor: TROIANO, Angelo, 6900 Lugano (CH); NICOLINI, Valentina, 6900 Lugano (CH); BELLORINI, Lorenzo, 6900 Lugano (CH); BERNAREGGI, Alberto, 6900 Lugano (CH); LUCHERINI, Enzo, 6900 Lugano (CH)
(74) Representative: Gerli, Paolo
(86) International application number: PCT/EP2014/001225
(87) International publication number: WO 2014/180565

(56) References cited:
- WO-A1-2012/158591
- FR-A1- 2 864 445
- JP-A- 2009 263 279
- PASTORE SAVERIA ET AL: "Plant Polyphenols Effectively Protect HaCaT Cells from Ultraviolet C-Triggered Necrosis and Suppress Inflammatory Chemokine Expression", NATURAL COMPOUNDS AND THEIR ROLE IN APOPTOTIC CELL SIGNALING PATHWAYS BLACKWELL PUBLISHING, 9600 GARSINGTON RD, OXFORD OX4 2DQ, OXEN, UK SERIES : ANNALS OF THE NEW YORK ACADEMY OF SCIENCES (ISSN 0077-8923(PRINT)), 2009, pages 305-313, XP008165985, & APOPTOSIS 2008 WORLD - FROM MECHANISMS TO APPLICATIONS; LUXEMBOURG, LUXEMBOURG; JANUARY 23 -26, 2008

## Description

### BACKGROUND ART

During the inflammatory process, the body produces important plasma- and cell-derived chemical mediators (histamine, interleukins, etc.) having reparative and/or amplification effects on the inflammatory phenomenon. The release and the activity of these mediators also generates oxygen free radicals (ROS, reactive oxygen species): these highly reactive species can cause damage to host cells, e.g. through the oxidation of cellular membranes and the alteration of their vital functions; the cellular structures thus damaged activate neutrophils and macrophages which, in their removal function, generate further oxygen radicals causing further damage, and amplifying the inflammatory process. Oxygen radicals may, for example, interact with vanilloid receptors of pain (TRVP1) and contribute to the activation of neurogenic inflammatory mechanisms and nociceptive response.

The development of oxygen free radicals is therefore an important part of the inflammatory process; the use of substances able to shield such species (radical scavengers) is generally desirable for the prevention and treatment of inflammatory conditions.

The majority of inflammatory diseases affect the skin and the mucosa; among them we may cite dermatitis, acne, psoriasis, ulcers, decubitus ulcers, phlebitis, hemorrhoids, vulvodynia, urethritis, etc.

In particular, **vulvodynia** is a common chronic vulvar condition characterized by pain, burning, irritation, redness or swelling sensation, dyspareunia, in the absence of objective visible alterations or specific clinically identifiable neurological disorders. The symptoms, either spontaneous or caused by contact, significantly interfere with the quality of life of women. The pain may be constant or intermittent, localized or diffused. The severity of the problem varies from mild discomfort to severe and debilitating pain. Vulvodynia may only affect the vaginal vestibule (vestibulodynia, approx. 80% of cases), the clitoral area (clitoridynia) or other areas of the vulva. In the generalized form, disorders affect a large part of the vulvar region (including perineum and anal region).

The three biological factors considered to be of greater importance in the onset of vulvodynia are an important vulvar vestibular inflammatory condition linked to the hyperactivation of mast cells, hypertonicity of the elevator muscle of anus of the myogenic type or acquired in response to a chronic vestibular inflammation and/or to pain, hyperactivity of the pain system.

Triggering causes may be repeated bacterial or viral vaginal infections (e.g. Candida, Escherichia coli, Chlamydia, Ureaplasma, Papillomavirus, Herpes Virus), phenomena of immediate hypersensitivity to environmental antigens, autoimmune diseases, effects of genital surgeries, mechanical microtrauma induced by sexual intercourse, tight clothing, vulvar pharmacological treatments, physical factors (laser and vulvar diathermocoagulation therapies, genital, urinary or anal radiotherapy), chemical factors (sugars and yeasts contained in certain foods, irritants in urine, use of aggressive detergents), hormonal factors (estrogenic fluctuations of the period, especially in the premenstrual phase), psycho-sexual (traumas, relationship conflicts) and neurogenic factors (the stimulus that induces the mast cell degranulation travels backwards through the sensory nervous ways, for example under stress conditions).

Anatomopathological studies have shown that in patients suffering from vulvodynia, vulvar and vestibular tissues have an inflammatory cellular infiltrate of T lymphocytes and mast cells.

The mast cell degranulation releases inflammatory mediators such as bradykinin, IL-1β, INF-1α, vasoactive factors, histamine, substance P, which cause vascular dilatation, redness, edema, heat and thus pain and local burning. Mast cells also release serotonin, an important neuromediator between mast cell and nerve fibers, neurotrophic substances such as NGF that multiply the nerve endings of pain making the sensations of hyperalgesia more intense. In addition to the proliferation of nerve endings, sensitization also occurs in vulvodynia, i.e. an increase in the responsiveness of thermoreceptors and nociceptors in the vestibular mucosa. In tissue samples from women with vulvodynia it was found an increase of the TRVP1 receptors, which can interact with the oxygen free radicals (13).

For the purposes of prevention of vulvodynia, different treatment approaches are used, often in combination, ranging from the use of medications, changes in diet (reduction of oxalate and calcium) and clothing (use of cotton underwear and comfortable clothing), psychotherapy, surgical therapy.

The pharmacological treatment of the disease includes the use of different classes of drugs for topical and/or systemic use. Among the topical preparations, creams and vestibular infiltrations of local anaesthetics (lidocaine) and corticosteroids (e.g. methylprednisolone and betamethasone) are used. Estrogens and tricyclic antidepressants (e.g. amitriptyline, desipramine), anticonvulsants (such as carbamazepine, gabapentin and pregabalin), selective serotonin reuptake inhibitors (SSRIs such as citalopram and paroxetine), norepinephrine reuptake inhibitors (SNRIs such venflaxine and duloxetine) and other therapies for the treatment of chronic neuropathic pain may be used in the systemic form.

An analysis of the factors involved in the onset of vulvodynia indicates that topical application of preparations effective in reducing the vulvar-vestibular inflammatory condition and the neuropathic pain to the vulvar area generates great benefits to patients suffering from this disease.

Urethritis is a pathological acute or chronic inflammatory process of the urethra. Urethritis can affect both females and males; the incidence of the disease is higher in males. Often, the inflammatory process also involves the bladder, particularly in women whose urethral duct is shorter than that of men. Urethritis may have an infectious or non-infectious origin. The latter is typically caused by trauma to the urethra, for example due to catheterization, frequent use of bicycle or motorcycle, the presence of kidney stones. The infectious forms are subdivided into *gonococcal urethritis* and *non-gonococcal urethritis.* Gonococcal urethritis (called *gonorrhea, blennorrhoea*) is the best known form of urethritis. Non-gonococcal urethritis accounts for about two-thirds of all urethritis. The micro-organisms that may cause non-gonococcal urethritis are quite numerous, but the vast majority of cases (about 80%) is caused by *Chlamydia trachomatis* and to a lesser extent by *Plasma urea uralyticum.* Non-gonococcal urethritis may cause infertility and, in the case of pregnancy, the presence of *Chlamydia* at the level of the uterine cervix could create some problems to the newborn. Non-gonococcal urethritisis is generally characterized by much milder symptoms than those of gonorrhea, with mild urethral secretions.

The person suffering from urethritis may experience burning, problems with urination, catarrhal or purulent secretions. In case of chronic infectious urethritis, a major complication is the possible spread of the infection to the closest structures; in men there may be cystitis, epididymitis, orchitis, pyelonephritis and prostatitis. Another complication is the narrowing of the urethral lumen resulting in the obstruction of the urinary flow. In women, there may be cervicitis, cystitis, ectopic pregnancy, miscarriage, pyelonephritis, salpingitis, etc. In males, urethritis may, extending to the testicles, cause infertility. In women it may be responsible for damage to the reproductive system and cause infertility.

The therapy of infectious urethritis is based on antibiotics. Regarding non-infectious urethritis, the treatment must tend to remove or otherwise manage the source that causes the irritative process.

Among the different methods of prevention and treatment of inflammatory diseases, it is known to use topical formulations (including creams, gels) containing anti-inflammatory substances with radical scavenging activity (*radical scavengers*); for some of these products, however, particularly in the case of macromolecules of plant origin, there is often little reproducible activity and a reduction in effectiveness over time. This fact represents an important limitation as opposed to the usefulness of developing natural medicaments. There is therefore the need of anti-inflammatory medicaments based on natural substances, which are highly effective and which maintain such a high activity over time.

The Applicant, active for years in the treatment of diseases of the genital and urinary systems, has done extensive research on specific substances of natural origin, including hyaluronic acid, verbascoside and inositol glycerophosphate.

Hyaluronic acid sodium salt (HANa) is a glycosaminoglycan, among the main components of the connective tissue, made of linear polymer chains of a disaccharide consisting of glucuronic acid and N-acetylglucosamine. The molecule is characterized by high polarity and, thanks to its high solubility in aqueous environment, ensures the hydration of the tissues at the same time protecting them from tension and stress. The viscous-elastic properties of hyaluronic acid provide plasticity to the tissues. Anti-inflammatory and healing properties of HANa are reported (1, 2). Among the various applications, it is known to use HANa in the treatment of interstitial cystitis.

Inositol glycerophosphate (GPI), also referred herein as glycerol-phospho-inositol, is an inhibitor of cytosolic phospholipase-A2 with cortisone-like activity. GPI induces inhibition of arachidonic acid release and consequently reduces the production of pro-inflammatory molecules (5).

Verbascoside (VB) is a natural polyphenolic compound, ester of caffeic acid, contained in plant species of the Lamiale order (Scrofulariaceae family), such as *Verbascum phlomoides* and *Verbascum mallophorum.* Verbascoside is also extracted from plant stem cells of Verbena Odorous (*Lippia Citriodora*) and Buddleja davidii grown in biofermentors on an industrial scale. Verbascoside is characterized by antimicrobial activity, in particular against *Staphylococcus aureus* (6) and possesses anti-inflammatory properties (7, 8).

Verbascoside inhibits the iNOS (inducible nitric oxide synthase) enzyme and acts as an antioxidant with *radical scavenger* properties towards reactive oxygen species (ROS) (9, 10). The verbascoside treatment of human THP-1 leukemia cells stimulated with pro-inflammatory stimuli (e.g. LPS and IFN-γ) in which the activity of iNOS is overexpressed, resulted in a significant decrease in the expression and activity of iNOS and superoxide radicals (7). In primary cultures of human keratinocytes stimulated with TNFα or IFN-γ, verbascoside reduces in a dose-dependent manner inflammatory chemokines such as GM-GCSF, IL-8, IP-10 and MCP-1. In vivo, verbascoside showed a marked anti-inflammatory effect in a periodontitis model in rats (8). The oral administration of verbascoside (2 mg/kg/day for 8 days) significantly reduces various parameters of inflammation and improves tissue damage. Analgesic activities of verbascoside in neuropathic pain were also reported (11).

### SUMMARY

The Applicant has surprisingly noted that HANa and GPI, substances per se devoid of antiradical activity, when associated to VB, increase the antiradical activity thereof in an unexpected way, thus obtaining an increased protective effect against inflammation. The use of the ternary composition of HANa, GPI or salt thereof, and VB as a pharmaceutical formulation with a high activity is therefore claimed for the treatment of inflammations of the skin and mucosa, in particular of vulvodynia and urethritis: the anti-inflammatory and analgesic efficacy was found in a clinical study conducted by the Applicant. The preferred ternary pharmaceutical forms are those in dry form (such as aspersion powders), or those in liquid or semi-solid form (creams, gels) which can be prepared prior to use by the addition of dry verbascoside to the remaining components.

### DESCRIPTION OF THE FIGURES

Figure 1: example of a single-dose, double chamber container for simultaneous delivery of two semi-solid forms kept separate from each other.
Figure 2: example of a multi-dose, double chamber container for simultaneous delivery of two preparations kept separate from each other.
Figure 3: examples of dispensers for muco-adhesive solution. 1: VB powder; 2: reservoir; 3 tank; 4: screw mouth closure; 5: ring-nut mouth closure; 6: dispenser; 7: solution of HA and GPI.

### DETAILED DESCRIPTION OF THE INVENTION

The invention is based on the ternary association of HANa, GPI and VB, the pharmaceutical compositions and kits containing said ternary association optionally in the presence of pharmaceutically acceptable excipients, the process of preparation thereof and their use in therapy.

In particular, the association is proposed for use in the prevention or treatment of inflammatory conditions of the skin and/or mucosa, especially of vulvodynia and other vulvar inflammatory conditions (e.g. vestibulitis), and male or female urethritis, particularly those of traumatic origin. The invention also includes the treatment of other inflammatory conditions affecting the mucosa and the skin, such as dermatitis, acne, psoriasis, ulcers, decubitus ulcers, phlebitis, hemorrhoids, etc.

Hyaluronic acid sodium salt (HANa) used in the invention can vary widely in its values of average molecular weight. A preferred range of average molecular weight is between 10 and 1000 kDa, more preferably between 10 and 500 kD, and optimally between 20 and 250 KDa.

Verbascoside is usable in all available forms. It may be used for example in pure form or in the form of plant extract obtained from a plant containing it, *such* as *Phlomoides Verbascum, Verbascum mallophorum, Lippia citriodora, Buddleja davidii* etc. or from plant stem cells from the same plants, grown on an industrial scale. It also possible to use verbascoside compositions mixed with suitable carriers, such as maltodextrins. Preferred commercial products are Dermasyr 50 and 10.

The GPI can be used as such, in form of a salt, e.g. choline salt; it can be used in solid form or as a solution at a concentration by weight, for example, between 5 and 20%.

The three active ingredients mentioned above can be used in variable ratios. By way of non-limiting example, taking 100% as the sum of HANa, VB and GPI (meant in the pure state, i.e. excluding the weight of possible liquid or solid carriers), they may be present in the following percentage ranges: 20-50% HANa, 40-60 GPI, 1-10% VB.

The formulations may also contain conventional excipients, depending on the specific pharmaceutical form made and on the desired use. Among them we may mention buffers, preservatives, gelling agents, bases for creams, emulsifiers, etc., as currently available in the pharmaceutical art.

The pharmaceutical forms contemplated by the invention are all those suitable to administer the medicament topically, or liquid, semi-solid or solid forms. Among the liquid forms are solutions, emulsions, colloids and suspensions; among the semi-solid forms are creams, ointments, gels; among the solid forms are lyophilized products and aspersion powders.

The Applicant has additionally observed that verbascoside is particularly stable upon storage when stored in the absence of water. Therefore, in a more preferred embodiment, the ternary association of the invention is formulated in dry form or with liquid or semi-solid anhydrous ingredients. If the final formulation requires one or more aqueous ingredients, verbascoside is preferably packaged separately in conditions of absence of water (or in the dry state or with semi-solid/liquid anhydrous ingredients) and stored in that form; a preferred semi-solid form is represented by glycerogel, or gelled semi-solid and non hydrated forms with a glycerin base; upon use, verbascoside is combined with the aqueous components, obtaining the final aqueous formulation, ready for administration. The separate packaging of the above ingredients is achievable by generally known means, e.g. double chamber bags, twin-bag double chamber dispensers, etc.

The invention therefore includes solid compositions comprising the three active ingredients of the invention in a physical mixture with one another, preferably in the absence of water. It further includes aqueous compositions comprising two separate pre-formulations to be mixed together prior to use, where the two formulations respectively consist of a verbascoside formulation and an aqueous of ingredient formulation. All extemporaneous formulations may be conveniently supplied to the user in the form of multi-component kit; such a kit may include means useful for performing the mixing of the formulations upon use, and instructions about how to do it. The extemporaneous mixing can be done manually by the user, or it can take place mechanically.

Particularly preferred forms for the purposes of the invention, and detailed in the examples, are the following:
1) Reconstitutable lyophilized form
2) Bi-component semi-solid form
3) Muco-adhesive solution
4) Aspersion powder

The bi-component composition of embodiment 2) refers to the above separation between VB and aqueous components. The muco-adhesiveness of embodiment 3) is guaranteed by the presence of HANa and any other muco-adhesive excipients. The aspersion powder characteristic of embodiment 4) is obtained by per se known techniques, including adequate micronization of the powder, the use of suitable excipients that increase smoothness, antiplatelet substances, the use of appropriate aspersion means, etc.

All the compositions of the invention can be in single-dose or multi-dose. They can also be provided with suitable dispensing means such as syringes, pumps, catheters, conventional and upside down spray dispensers, etc.

A preferred composition is a single-dose bi-component semi-solid form in which the verbascoside, preferably formulated as a glycerogel, is packaged separately from an aqueous-base cream containing hyaluronic acid sodium salt and glycero-phospho-inositol, reconstitutable upon use.

A further preferred composition is a form in which the physical separation of verbascoside, preferably formulated as a glycerogel, and of the cream is ensured by the use of single-dose double-chamber sachets. Upon use, verbascoside and cream are simultaneously dispensed from the respective chambers of the containers and manually homogenized. The resulting semi-solid mixture, with pH ranging from 3 to 7, is then applied on the affected part.

A further preferred composition is a multi-dose bi-component semi-solid form in which the verbascoside, preferably formulated as a glycerogel, is packaged separately from an aqueous-base cream containing hyaluronic acid sodium salt and glycero-phospho-inositol, reconstitutable upon use. The physical separation of the verbascoside and cream can be ensured by the use of a multi-dose double-chamber dispenser. Upon use, verbascoside and cream are simultaneously dispensed from the respective chambers of the containers and manually homogenized. The resulting semi-solid mixture, with pH ranging from 3 to 7, is then applied on the affected part.

A further preferred composition is a multi-dose muco-adhesive solution. It may be contained in a bottle of plastic or glass provided with a cap-tank which contains verbascoside in solid form. This ensures the physical separation of incompatible components until use. The composition is reconstitutable by slight pressure exerted on the cap that allows the breaking of a partition and the fall of the verbascoside powder in the solution of hyaluronic acid sodium salt and glycero-phospho-inositol. A subsequent short mixing enables rapid solubilization of the powder to generate the ready to use solution with pH ranging from 3 to 5. The solution can be dispensed by a pump device (semisolid dispenser or conventional or upside down spray), with or without cannula, for the on-site application of the preparation.

A further preferred composition is a formulation in the form of powder obtained by direct mixing of the powders of hyaluronic acid sodium salt, verbascoside and glycero-phospho-inositol or by grinding/micronization of a corresponding lyophilized product, dispensable via suitable device.

The invention extends to a process of preparation of the above compositions or kits, characterized by the use of the three active ingredients mentioned above, which are formulated in solid, semi-solid or liquid form, together or separately, as described above, depending on the desired final formulation.

The following experimental part shows in a non-limiting manner the biological and clinical results obtained with the combination of the invention, and the preparation of the pharmaceutical compositions useful to carry out the proposed treatment.

### Radical scavenging activity of HANa-, VB- and GPI based preparations

Evidence of the radical scavenging activity of the main ingredients of the formulations prepared, VB, HANa, GPI, were obtained with *in vitro* tests.

The anti-radical activity of VB alone and in combination with HANa and GPI was analyzed by spectrophotometric test with 2,2-diphenyl-1-picrylhydrazyl (DPPH). The samples were prepared respecting the proportions provided in the formulation. The activity of the compound(s) in question is shown in Tables 1 and 2:

**Table 1**

| **VB concentration (µM)** | **VB concentration (mg/L)** | **Radical scavenging activity (%)** |
|---|---|---|
| 0 | 0 | 0 |
| 53 | 33.1 | 95 |
| 26.5 | 16.6 | 76 |
| 13.2 | 8.3 | 42 |
| 6.6 | 4.1 | 24 |
| 3.3 | 2.1 | 15 |

**Table 2**

| **Compound** | **Concentration (mg/L)** | **Radical scavenging activity (%)** |
|---|---|---|
| | 0 | 0 |
| VB | 15.6 | 74 |
| HANa | 62.4 | 1 |
| GPI | 78 | 1 |
| HANa + VB | 62.4 + 15.6 | 85 |
| GPI + VB | 78 + 15.6 | 83 |
| GPI + HANa | 78 + 62.4 | 1 |
| GPI + HANa + VB | 78 + 62.4 + 15.6 | 95 |

The radical scavenging activity of VB alone is clear. HANa and GPI alone essentially showed no activity. However, in combination with VB, these components increase the radical scavenging effect of VB. It should be noted that oxygen radicals may (ROS, reactive oxygen species) may interact with vanilloid receptors of pain (TRVP1) and contribute to the activation of neurogenic inflammatory mechanisms and nociceptive response (12). An increase in TRVP1 receptors (13) was observed in tissue samples from women with vulvodynia. Therefore, the co-presence in situ of VB, HANa and GPI creates the conditions for a synergistic action that leads to the reduction of neuropathy and pain that occur in patients suffering from vulvodynia. The stable formulations obtained have confirmed the analgesic activity of the preparation in a preliminary study in patients with vulvodynia.

### Preliminary clinical studies for the treatment of vulvodynia

Preliminary clinical trials of efficacy and tolerability were performed in 10 patients with acute vulvodynia and dyspareunia with cutaneous dysesthesia with burning and peri-intra-vaginal and genital itching. The subjects treated 3 times a day with the formulation shown in Example 9, described below, have reported remission of symptoms in 5-7 days. The treated subjects experienced an immediate local desensitizing action with the disappearance of: pain, burning, dryness and itching. Each patient reported immediate and excellent feeling of well-being, hydration and lubrication. The well-being, initially 4-5 hours, then extended up to 12 hours. After one week, all patients reduced the application to 2 times, morning and evening, then gradually discontinued therapy.

The disappearance of redness and fissures was observed locally. The fissures, if any, disappeared within 10 days with restoration of the normal tissue, an evidence of a healing action of the association of the three characteristic components of the composition object of the present invention.

### Preparation of reconstitutable lyophilized forms

In this preparation the three components VB, HANa and GPI are combined into a single solid phase. A fresh solution containing VB, HANa, GPI, excipients and preservatives is lyophilized in a suitable bottle, sealed at the end of the process. After reconstitution with purified or higher grade water, ring nut and cap are removed, a dispenser pump is introduced into the bottle and the preparation is ready for use. The product can be packaged using type I white glass bottles, in dark glass, or using modern bottles made of transparent plastic (COC - cyclo-olefinic copolymers type). The final package is prepared by coupling the bottle with a standard horizontal conical atomizer pump applicable by pressure (or possibly screw); for the purpose of a more convenient application, pumps with "upside down" technology as well as adjustable may be used, with typical turnable cannula to allow dispensing of the product even with upside down dispenser; the dispensing capacity of the pump may be for example 100 µL/spray, being however selected according to the dose.

Examples 1-3 describe, methods of preparation of such formulations.

### EXAMPLE 1

The lyophilized form is made from: HANa MW 50 - 250 kDa (0.21%), an aqueous solution at 10% of GPI choline salt (2.5%), Dermasyr 50 (VB: maltodextrins, 1:1) (0.1%), methylparaben (Nipagin) (0.2%), propylparaben (Nipasol) (0.02%), sodium dihydrogen phosphate dihydrate (0.78%), phosphoric acid (0.093%), ascorbic acid (0.10%), water (as needed at 100%).

To this end, 479.985 g water are poured in a glass beaker. At room temperature, in a sequence and under continuous mechanical stirring at a speed of 400 RPM, 1.05 g HANa, 12.5 g GPI choline salt 10%, 0.25 g VB, 0.25 g maltodextrin, 1.0 g Nipagin, 0.1 g Nipasol, 3.9 g sodium dihydrogen phosphate dihydrate, 0.465 g phosphoric acid and 0.5 g ascorbic acid are introduced. The complete dissolving of the component added is waited for between one addition and the subsequent one.

The resulting solution is divided into aliquots of 10 g in type I white glass bottles.

The prototype is prepared with the aid of a laboratory lyostat.

The lyophilization cycle comprises a step of freezing at -40 °C, a step of primary heating at -10 °C and subsequent heating steps at 6 °C, 12 °C, 16 °C and 17.5 °C; unloading is carried out when the temperature measured for the product is around the room temperature and the vacuum in the chamber does not undergo variations (meaning that the sublimation process has ended). The cycle end involves the stoppering activity with slight return of nitrogen, in depression. The cap used is made of butyl rubber. The bottles, removed from the lyophilization chamber, are immediately sealed with aluminum flip-off ring nut cap.

The lyophilized product is reconstituted with 10 mL purified water. The reconstitution takes place under mild stirring of the bottle over 30 seconds. A clear solution is obtained, with pH of 4.7, slightly viscous (viscosity of about 5 mPas s⁻¹) and muco-adhesive, useful for the permanence of VB, HANa and GPI on the administration site. The data collected following a forced stability, conducted for 20 days at 40 "C-75% R.H. showed excellent quality of lyophilized form. Even after reconstitution with water, the chemical, physical and microbiological stability of the reconstituted formulation, stored at room temperature, is ensured for over 5 days, and this allows a normal treatment cycle to be carried out having full guarantee of a stable and reproducible effect.

The pump used for dispensing is an "upside down" with a six cm cannula with 100 µL per spray.

### EXAMPLE 2

This example reproduces the composition of Example 1, but without ascorbic acid. Also this composition is characterized by chemical, physical and microbiological stability when subjected to forced degradation conditions (40 °C-75% R.H. for 20 days), of a degree equivalent to that of Example 1.

The formulation comprises HANa MW 50 - 250 kDa (0.21%), an aqueous solution at 10% of GPI choline salt (2.5%), Dermasyr 50 (VB: maltodextrins, 1:1) (0.1%), methylparaben (Nipagin) (0.2%), propylparaben (Nipasol) (0.02%), sodium dihydrogen phosphate dihydrate (0.78%), phosphoric acid (0.093%), water (as needed to 100%). To this end, 480.485 g water are poured in a glass beaker. At room temperature, in a sequence and under continuous mechanical stirring at a speed of 400 RPM, 1.05 g HANa, 12.5 g GPI choline salt 10%, 0.25 g VB, 0.25 g maltodextrin, 1.0 g Nipagin, 0.1 g Nipasol, 3.9 g sodium dihydrogen phosphate dihydrate and 0.465 g phosphoric acid are introduced. The complete dissolving of the component added is waited for between one addition and the subsequent one.

The resulting solution is subdivided into aliquots of 10 g in type I white glass bottles.

The prototype is prepared with the aid of a laboratory lyostat.

The lyophilization cycle comprises a step of freezing at -40 °C, a step of primary heating at -10 °C and subsequent heating steps at 6 °C, 12 °C, 16 °C and 17.5 °C; unloading is carried out when the temperature measured for the product is around the room temperature and the vacuum in the chamber does not undergo variations (meaning that the sublimation process has ended). The cycle end involves the stoppering activity with slight return of nitrogen, in depression. The cap used is made of butyl rubber. The bottles, removed from the lyophilization chamber, are immediately sealed with aluminum flip-off ring nut cap.

The lyophilized product is reconstituted with 10 mL reconstituting solution generated with purified water. The reconstitution takes place under mild stirring of the bottle over 30 seconds.

The resulting solution, stored at room temperature, maintains optimum chemical, physical and microbiological stability for over 5 days, even during use.

The pump used is an "upside down" with a six cm cannula with 100 µL per spray.

### EXAMPLE 3

Lyophilized form without ascorbic acid as antioxidant, preservative-free, characterized by chemical, physical and microbiological stability when subjected to forced degradation conditions (40 °C-75% R.H. for 20 days).

The formulation comprises HANa MW 50 - 250 kDa (0.21%), an aqueous solution at 10% of GPI choline salt (2.5%), Dermasyr 50 (VB: maltodextrins, 1:1) (0.1%), sodium dihydrogen phosphate dihydrate (0.78%), phosphoric acid (0.093%), water (as needed to 100%).

481.585 g water are poured in a glass beaker; at room temperature, in a sequence and under continuous mechanical stirring at a speed of 400 RPM, the following are added: 1.05 g HANa, 12.5 g GPI choline salt 10%, 0.25 g VB, 0.25 g maltodextrin, 3.9 g sodium dihydrogen phosphate dihydrate and 0.465 g phosphoric acid. The complete dissolving of the component added is waited for between one addition and the subsequent one.

The resulting solution is subdivided into aliquots of 10 g in type I white glass bottles.

The prototype is prepared with the aid of a laboratory lyostat.

The lyophilization cycle comprises a step of freezing at -40 °C, a step of primary heating at -10 °C and subsequent heating steps at 6 °C, 12 °C, 16 °C and 17.5 °C; unloading is carried out when the temperature measured for the product is around the room temperature and the vacuum in the chamber does not undergo variations (meaning that the sublimation process has ended). The cycle end involves the stoppering activity with slight return of nitrogen, in depression. The cap used is made of butyl rubber. The bottles, removed from the lyophilization chamber, are immediately sealed with aluminum flip-off ring nut cap.

The lyophilized product is reconstituted with 10 mL reconstituting solution generated with purified water. The reconstitution takes place under mild stirring of the bottle over 30 seconds.

The resulting solution, if stored at room temperature, maintains optimum chemical, physical and microbiological stability for at least 5 days, even during use.

The pump used is an "upside down" with a six cm cannula with 100 µL per spray.

### Preparation of single-dose and multi-dose semi-solid bi-component forms

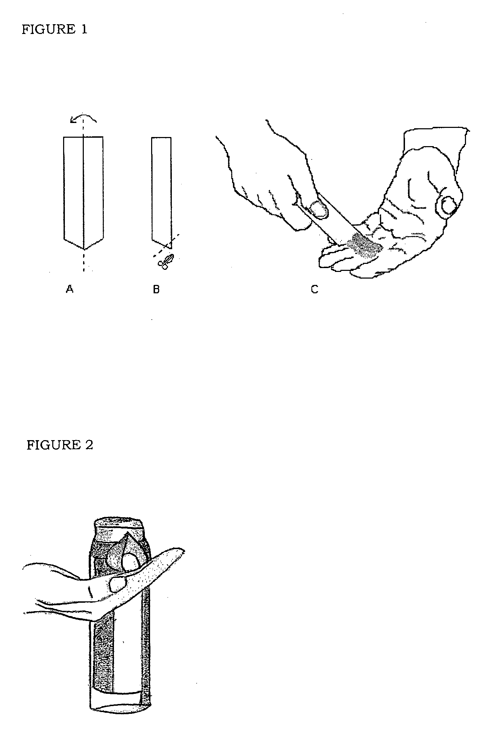

In this preparation a gel, e.g. a glycerogel containing VB, is kept physically separated from an aqueous-based cream containing HANa and GPI. The physical separation of glycerogel and cream is ensurable, until use, by partitioning systems currently used in the pharmaceutical art. For example, single-dose dual-chamber sachets (Figure no. 1) or a multi-dose double-tube dispenser (Figure no. 2) may be used. Upon use, the glycerogel and cream preparations are simultaneously dispensed from the respective tanks of the containers and manually homogenized. The resulting semi-solid mixture is then applied on the affected part.

Examples 4-5 show the preparation of said embodiments according to the invention.

### EXAMPLE 4

This is a semi-solid pharmaceutical form consisting of two preparations mixable upon use.

### A) Preparation of glycerogel containing VB.

The glycerogel has the following percentage composition: Dermasyr 50 (VB: maltodextrins, 1:1) (0.2%), anhydrous glycerin 1.26 (75%), propylene glycol (23.3%), Sepineo® P 600 (1.5%). The latter component is a concentrated dispersion of an acrylamide copolymer with self-gelling properties, i.e. sodium acryloyldimethyltaurate, in isohexadecane.

The preparation takes place at room temperature (25 + 5 °C).

1875.0 g pure anhydrous glycerin 1.26 are added to a three-liter beaker and, under mechanical stirring at 500 RPM, 2.5 g VB and 2.5 g maltodextrin are added; stirring is maintained until complete dissolution of the powders in the glycerin.

Reducing the stirring to 100-200 RPM, 582.5 g propylene glycol are added to the solution and stirring is continued up to complete mixing.

The final processing step involves the addition of the remaining 37.5 g Sepineo P 600 which, under stirring at 500 RPM continued for thirty minutes after the formation of the gel, allows an even, clear yellowish-beige preparation to be made, characterized by a viscosity, at 25 °C, of 7500 mPas s⁻¹.

### B) Preparation of the cream containing HANa and GPI.

The cream has the following percentage composition: HANa (0.56%), aqueous solution of GPI choline salt at 10% (6.21%), Sepineo® SE 68 (alkyl polyglucoside) (10.0%), Cetiol® V (decyl oleate) (5.0% ), Lanette® SX (cetearyl alcohol and sodium lauryl sulfate) (2.0%) pure anhydrous glycerin 1.26 (1.5%), sorbitol 70% (1.5%), sorbic acid (0.15%), methylparaben (0.2%), propylparaben (0.02%), sodium dihydrogen phosphate dihydrate (0.6%), ultra-filtered water (72.26%).

The following method of preparation describes how to make 2500 g cream.

250 g Sepineo® SE 68, 50 g Lanette® SX, 125 g Cetiol® V, 3.75 g sorbic acid, 5 g methylparaben and 0.5 g propylparaben are added to a first 3-liter beaker. The container is placed in bain-marie at a temperature of 65-70 °C in order to melt the above components. The fat phase of the preparation is thus obtained.

1306.48 g purified water are added to a second 3-liter beaker. This is heated to 65-70 °C on a heating plate equipped with a magnetic stirrer. A small aliquot of 50 g is taken in order to carry out final rinsing, then 37.5 g pure anhydrous glycerin 1.26, and 37.5 g sorbitol 70% are added at hot, up to the total dissolution of the two components at the above temperature under continuous and regular stirring (200-300 RPM).

The remaining part of ultra filtered water (500 g) is poured into a third 1-liter beaker placed on a magnetic stirrer. The water is heated to 65-70 °C, 14.02 g HANa are added and stirred at 200-300 RPM up to complete dissolution of the added powder.

The solution thus obtained is poured into the second beaker prepared previously and homogenization is carried out at a stirring speed of 200-300 RPM.

155.25 g aqueous solution of GPI choline salt at 10% are then added. The phase is completed by dissolving 15 g sodium dihydrogen phosphate dihydrate in the solution. The aqueous phase of the preparation is thus obtained.

A turboemulsifier is immersed in the first beaker containing the fat phase of the cream, the turbine is operated at low speed and the totality of the aqueous phase contained in the second beaker is added, carrying out a rinsing with 50 g ultra filtered water set aside at the beginning of the processing. Simultaneously with the addition of the aqueous phase, the turbine is brought to a speed of 7000 RPM leaving it inserted for 5 minutes. The resulting cream is cooled under mechanical stirring (300-400 RPM) until it reaches a temperature below 30 °C.

The pH of this preparation is around 4.5 and viscosity at 25 °C is 909 mPas s⁻¹.

The analysis of the titratable components added to the two preparations gave the following results (Table 3)

**Table 3**

| **COMPONENT** | **% TITRE** |
|---|---|
| Sorbic acid | 99.1 |
| Methylparaben | 103.2 |
| Nipasol | 102.4 |
| Hyaluronic acid | 104.4 |
| Glycero-phospho-inositol (GPI) | 99.8 |
| Verbascoside | 99.8 |

The two topical semi-solid compositions produced by the above-described procedures are dispensed by means of a single-dose double chamber container (figure no. 1).

The sachet in this example is prepared with a multi-layered film commonly used for storing this type of semi-solid formulations. In particular, a film is used which consists of the superposition of polyethylene terephthalate layers (PET, 12 µm), a dye (1 µm), adhesive (3.5 µm), aluminum (Al, 12 µm), adhesive (2 µm) and polyethylene (PE, 45 µm), for a total of 75.5 µm. Films with a thicknesses up to a maximum of 200 µm were considered, which allow easy handling and squeezing of the product. Al, PVC (polyvinyl chloride), PE, PVDC (polyvinylidene chloride), PET, PP (polypropylene), EVOH (ethylene vinyl alcohol), Aclar®, Surlyn®, nylon, polystyrene or other suitable materials and variable combinations and stratification thereof are the materials employable to this end.

The peculiar form devised allows two semi-solid preparations to be delivered via a single squeezing action. The two separate chambers of the sachet are identical in shape and are generated by hot moulding in a manner symmetrical with respect to the vertical axis of the object (Figure 1A). A common volumetric dosing device for creams/gels in "sachet" guarantees the proper filling of the above chambers so that these can be hermetically sealed by heat sealing and pressure. The allocable volumes depend on the useful capacity obtained for the single chamber. The particular softness of the device allows the total squeezing of the preparation ensuring accurate doses. The shapes can be chosen so that the dispensing may be easy and total. In Figure 1, the selected shape is a rectangle that ends in a triangle; overlaying a chamber on its symmetric it will be possible to cut the resulting angle (Figure 1B). A slight and continuous pressure will allow the dispensing of the two formulations to be applied simultaneously on the palm of the hand (Figure 1C), or directly on the affected area. This type of packaging is intuitively usable for all those preparations that need to be separated from each other for reasons of physical or chemical incompatibility, but the effective use thereof must be simultaneous and/or synergistic.

In the case described above, each chamber of the sachet described dispenses the amount of 0.6 g of the preparation for a total of 1.2 g.

The pH of the final mixture is equal to 4.54 with a viscosity of 1190 mPas s⁻¹.

### EXAMPLE 5

The two semi-solid compositions of glycerogel and cream are made according to the preparation method described in Example 4. The dispensing of glycerogel and cream this time is done by means of a dual chamber container with two multi-dose full-emptying reservoirs communicating with two orifices in the cap (Figure 2). Each of the two reservoirs of the bottle is filled with 30 mL of the preparation, i.e. with 35 g glycerogel and 27 g cream, respectively, differing in density. Following a single pressure of the cap, each orifice in the dispenser dispenses 0.96 mL preparation for a total of 1.92 mL, consisting of 1.1 g glycerogel and 0.74 g cream.

The pH of the final mixture is equal to 4.54 and viscosity is 1190 mPas s⁻¹.

The preparations in Examples 4 and 5 were subjected to accelerated stability tests conducted for 20 days at 40 °C-75% R.H. and for 2 months at 25 °C-60% R.H. The data collected showed the stability of the two preparations.

### Preparation of multi-dose muco-adhesive solutions

In this preparation, the muco-adhesive solution of HANa and GPI is contained, together with suitable excipients, in a bottle of plastic or glass. The bottle is sealed with a partition adhering to the mouth to avoid accidental spills of the preparation. The VB powder in solid form (powder with suitable excipients to ensure support, storage and flow of the powder itself) can be divided into a sachet or in a reservoir-cap. The reconstitution can be done in 2 ways depending on the powder container selected.

If the VB powder is contained in the sachet, this is cut at an end and the content is poured inside the bottle.

If the VB powder is divided in the reservoir-cap of the bottle, a slight pressure is exerted on the reservoir-cap that allows for the breaking of a plastic partition and this allows the fall of the VB powder in the HANa and GPI solution.

In both cases, mixing is advisable to obtain the solubilization of the VB powder to produce a ready-to-use solution. The bottle is then opened and a suitable dispenser suitable for the on-site dispensing of the preparation is applied by pressure or screw-wise.

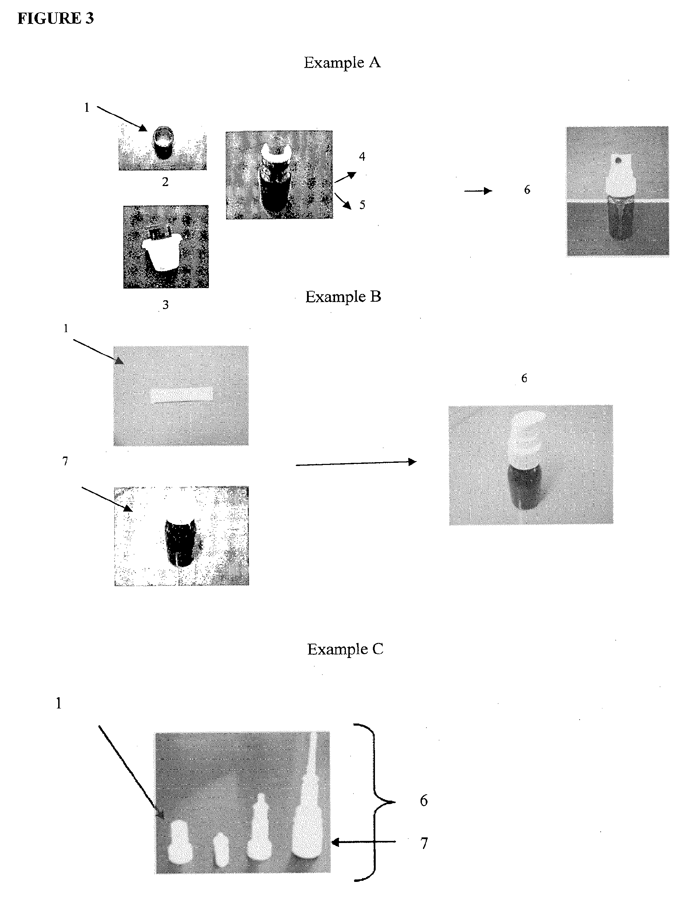

Various examples of dispensers for the muco-adhesive solution are shown in Figure 3.

Specific preparations of muco-adhesive solutions are described in Examples 6-9.

### EXAMPLE 6

The case described deals with the preparation of a muco-adhesive solution at pH 4.4 and viscosity of 60 mPas s⁻¹ at 25 °C, containing HANa (2.08), a solution of GPI choline salt at 10% (24.75%), ultra filtered water (41.58%), propylene glycol (29.7%), sodium dihydrogen phosphate dihydrate (0.79%) and phosphoric acid 85% (0.1%). Dry Dermasyr 50 is added to the reservoir in an amount of 1% w/w on a total of 10 g muco-adhesive solution.

1200 g of the solution containing HANa and GPI are prepared in a 2-liter beaker.

360 g propylene glycol, 493.2 ultra filtered water and 300 g GPI choline salt 10% are weighed in the beaker. Solubilization is done by magnetic stirring at a speed of 300-400 RPM at 60 °C. 25.2 g HANa are added to the resulting preparation and the solution is completely solubilized; then, 9.6 g sodium dihydrogen phosphate dihydrate are added and completely solubilized. Cool the solution to the temperature of 25 °C under continuous mild stirring. The pH must now be brought to the value of 4.4 by adding 1.2 g phosphoric acid 85% diluted in 10.8 g ultra filtered water. The solution is then ready for being subdivided.

The solution (9.9 g) is subdivided into brown glass bottles of appropriate capacity. The reservoir-cap is filled with 100 mg Dermasyr 50 powder (50 mg VB and 50 mg maltodextrin).

Upon use, after the dissolution of the powder in solution, an "upside down" pump is mounted with a six-cm cannula capable of dispensing 100 mg solution per dispensing.

The analysis of the solution gave the results shown in Table 4:

**Table 4**

| **COMPONENT** | **Initial % titre** | **% titre at 30 days at room temperature** |
|---|---|---|
| Hyaluronic acid | 101.3 | 100.7 |
| Glycero-phospho-inositol (GPI) | 99.5 | 98.2 |
| Verbascoside | 99.6 | 92.5 |

### EXAMPLE 7

Preparation of a muco-adhesive solution with pH 4.4 and viscosity of 145 mPas s⁻¹ at 25 °C containing HaNa (2.08%), GPI choline salt 10% (25%) (24.75%), ultra filtered water (41.34%), propylene glycol (29.7%), sodium dihydrogen phosphate dihydrate (0.79%), methyl paraben (0.2%), phosphoric acid 85% (0.1%), propyl paraben (0.02%) and BHA (0.02%), Dry Dermasyr 50 is added to the reservoir-cap in an amount of 1% w/w.

1200 g of the solution containing HANa and GPI are prepared in a 2-liter beaker. 360 g propylene glycol, 490.32 ultra filtered water and 300 g GPI choline salt 10% are weighed in the beaker. The solution is solubilized under magnetic stirring (at a speed of 300-400 RPM) at 60 °C.

25.2 grams HaNa are added to the resulting preparation and the process is continued up to their complete dissolution. 9.6 g sodium dihydrogen phosphate dihydrate, 2.4 g methyl paraben, 0.24 g propyl paraben and 0.24 g BHA are added and solubilized. The solution is cooled to the temperature of 25 °C under continuous mild stirring. The pH is brought to 4.4 by adding 1.2 g phosphoric acid 85% diluted in 10.8 g ultra filtered water. The solution is then ready for being subdivided.

The solution (9.9 g) is subdivided into brown glass bottles of appropriate capacity. The reservoir-cap is filled with 100 mg Dermasyr 50 powder (50 mg VB and 50 mg maltodextrin).

Upon use, after the dissolution of the powder in solution, an "upside down" pump is mounted with a six-cm cannula capable of dispensing 100 mg solution per dispensing.

The analysis of the solution gave the results shown in Table 5.

**Table 5**

| **COMPONENT** | **Initial % titre** | **% titre at 10 days at room temperature** |
|---|---|---|
| Hyaluronic acid | 99.32 | 96.26 |
| Glycero-phospho-inositol (GPI) | 99.63 | 102.88 |
| Verbascoside | 101.68 | 98.71 |

### EXAMPLE 8

Preparation of a muco-adhesive solution at pH 4.4 and viscosity of 60 mPas s⁻¹ at 25 °C containing HaNa (2.08%), GPI choline salt 10% (24.75%) (24.75%), ultra filtered water (41.56%), propylene glycol (29.70%), sodium dihydrogen phosphate dihydrate (0.79%), phosphoric acid 85% (0.1%) and BHA (0.02%). Dry Dermasyr 50 is added to the reservoir in an amount of 1% w/w.

1200 g of the solution containing HANa and GPI are prepared in a 2-liter beaker. 360 g propylene glycol, 492.96 ultra filtered water and 300 g GPI choline salt 10% are weighed in the beaker. The solution is solubilized under magnetic stirring (at a speed of 300-400 RPM) at 60 °C.

25.2 grams HaNa are added to the resulting preparation. After solubilisation, 9.6 g sodium dihydrogen phosphate dihydrate and 0.24 g BHA are added and dissolved. The solution is cooled to the temperature of 25 °C under continuous mild stirring. The pH is brought to 4.4 by adding 1.2 g phosphoric acid 85% diluted in 10.8 g ultra filtered water. The solution is then ready for being subdivided.

The solution (9.9 g) is subdivided into brown glass bottles of appropriate capacity. The reservoir-cap is filled with 100 mg Dermasyr 50 powder (50 mg VB and 50 mg maltodextrin).

Upon use, after the dissolution of the powder in solution, an "upside down" pump is mounted with a six-cm cannula capable of dispensing 100 mg solution per dispensing.

The analysis of the solution gave the results shown in Table 6.

**Table 6**

| **COMPONENT** | **Initial % titre** | **% titre at 10 days at room temperature** |
|---|---|---|
| Hyaluronic acid | 98.48 | 96.26 |
| Glycero-phospho-inositol (GPI) | 98.17 | 102.24 |
| Verbascoside | 102.2 | 98.55 |

### EXAMPLE 9

Preparation of a muco-adhesive solution at pH 4.3 and viscosity of 60 mPas s⁻¹ at 25 °C containing HANa (0.42%), GPI choline salt solution 10% (5%) (5%), ultra filtered water (57.7%), propylene glycol (29.94%), anhydrous glycerine 1.26 (5,0%), iota carrageenan (0.9%), sodium dihydrogen phosphate dihydrate (0.8%) and phosphoric acid 85% (0.04%) Dry Dermasyr 50 is added to the sachet in an amount of 0.2% w/w.

1200 g of the solution containing HANa and GPI are prepared in a 2-liter beaker. 360 g propylene glycol, 60 g anhydrous glycerine 1.26, 658.02 ultra filtered water and 60 g GPI choline salt 10% are weighed in the beaker. It is solubilized under magnetic stirring (at a speed of 300-400 RPM) at 60 °C. 5.04 g HANa are solubilized in the resulting preparation. The solution is cooled to the temperature of 25 °C under continuous mild stirring. 10.8 g iota carrageenan are then added and solubilized. 9.6 g sodium dihydrogen phosphate dihydrate previously solubilized are then added to 24 g deionized water. The pH is brought to 4.3 by adding 0.54 g phosphoric acid 85% previously diluted in 12.0 g ultra filtered water. The solution is then ready for being subdivided.

The solution (34.93 g) is subdivided into plastic bottles of appropriate capacity. The sachet described above is filled with 70 mg Dermasyr 50 powder (35 mg VB and 35 mg maltodextrin). Upon use, after the dissolution of the powder in solution, a dispenser is mounted capable of dispensing 0.5 g solution per dispensing.

The analysis of the solution gave the results shown in Table 7.

**Table 7**

| **COMPONENT** | **Initial % titre** | **% titre at 30 days at room temperature** |
|---|---|---|
| Hyaluronic acid | 114.6 | 114.9 |
| Glycero-phospho-inositol (GPI) | 100.6 | 100.8 |
| Verbascoside | 101.7 | 92.9 |

The data shown in Tables 4-7 are of interest especially with reference to verbascoside, since this component is particularly labile in an aqueous medium. The tables show that the VB, after extemporaneous incorporation with the aqueous component according to the present invention, still maintains a high titre for at least 30 days at room temperature, thus allowing the completion of one or more treatment cycles and ensuring a high and reproducible effect.

### Preparation of aspersion powder

This preparation is obtained after solubilization of HANa, VB and GPI and subsequent lyophilization followed by micronization to obtain an average particle size of 5-15 µm. This powder added with suitable excipients is dispensed with the use of a suitable container.

### BIBLIOGRAPHY

1. M.G. Neuman, R.M. Nanau, L. Oruna, G. Coto. In vitro Anti-Inflammatory Effects of Hyaluronic Acid in Ethanol-Induced Damage in Skin Cells. J Pharm Pharmaceut Sci, 14(3) 425 - 437, 2011
2. Weigel PH, Frost SJ, McGary CT, LeBoeuf RD. The role of hyaluronic acid in inflammation and wound healing. Int J Tiss React 1988;10:355-65.
3. Peters K, Girdler B, Carrico D, Ibrahim I, Diokno A. Painful bladder syndrome/interstitial cystitis and vulvodynia: a clinical correlation. Int Urogynecol J Pelvic Floor Dysfunct. 2008, 19(5):665-9.
4. Carrico DJ, Sherer KL, Peters KM. Urol Nurs. The relationship of interstitial cystitis/painful bladder syndrome to vulvodynia, 2009, 29(4):233-8.
5. Cucullo L, Hallene K, Dini G, Dal Toso R, Janigro D. Glycerophosphoinositol and dexamethasone improve transendothelial electrical resistance in an in vitro study of the blood-brain barrier. Brain Res. 2004, 6;997(2):147-51.
6. Avila JG, de Liverant JG, Martinez A, Martinez G, Munoz JL, Arciniegas A, Romo de Vivar A. Mode of action of Buddleja cordata verbascoside against Staphylococcus aureus. Journal of Ethnopharmacology, Volume 66, 1, 75-78, 1999.
7. Speranza L, Franceschelli S, Pesce M, Reale M, Menghini L, Vinciguerra I, De Lutiis MA, Felaco M, Grilli A. Antiinflammatory effects in THP-1 cells treated with verbascoside. Phytother Res. 2010 Sep;24(9):1398-404.
8. Di Paola R, Oteri G, Mazzon E, Crisafulli C, Galuppo M, Dal Toso R, Pressi G, Cordasco G, Cuzzocrea S. Effects of verbascoside, biotechnologically purified by Syringa vulgaris plant cell cultures, in a rodent model of periodontitis. J. Pharmacy and Pharmacology 2011, 63, 707-717.
9. Esposito E, Dal Toso R, Pressi G, Bramanti P, Meli R, Cuzzocrea S. Protective effect of verbascoside in activated C6 glioma cells: possible molecular mechanisms. Naunyn Schmiedebergs Arch Pharmacol. 2010, 381(1):93-105
10. Gao JJ, Igalashi K, Nukina M. Radical scavenging activity of phenylpropanoid glycosides in Caryopteris incana. Biosci Biotechnol Biochem. 1999, 63(6):983-8.
11. Isacchi B, Iacopi R, Bergonzi MC, Ghelardini C, Galeotti N, Norcini M, Vivoli E, Vincieri FF, Bilia AR. Antihyperalgesic activity of verbascoside in two models of neuropathic pain. J Pharm Pharmacol. 2011 63(4):594-601.
12. Westlund KN, Kochukov MY, Lu Y, McNearney TA. Impact of central and peripheral TRPV1 and ROS levels on proinflammatory mediators and nociceptive behavior. Mol Pain. 2010, 6;6:46.
13. Tympanidis P, Casula MA, Yiangou Y, Terenghi G, Dowd P, Anand P. Increased vanilloid receptor VB1 innervation in vulvodynia. Eur J Pain. 2004;8(2): 129-33.

## Claims

1. Association of hyaluronic acid sodium salt (HANa), verbascoside (VB) and glycero-phospho-inositol (GPI).

2. Association according to claim 1 for use in therapy.

3. Association according to claims 1-2 for topical use in the prevention or treatment of inflammatory and/or painful conditions of the skin and/or mucosa.

4. Association according to claims 1-3, for use in the prevention or treatment of inflammatory and/or painful conditions of the female and male genital apparatuses, in particular vulvodynia, vestibulitis, urethritis, or for other inflammatory conditions such as ulcers, decubitus ulcers, phlebitis, dermatitis, acne, psoriasis, hemorrhoids.

5. Stable pharmaceutical composition comprising the association according to claim 1, in presence of one or more pharmaceutically acceptable excipients.

6. Composition according to claim 5, free from aqueous ingredients.

7. Composition according to claim 5, comprising aqueous ingredients, in which the verbascoside is packaged separately from said aqueous ingredients.

8. Composition according to claims 5-7, in solid, semi-solid or liquid form; mono-dose or multi-dose.

9. Composition according to claims 5-7, formulated as: liophilized form, semi-solid bi-component form, muco-adhesive solution, or powder.

10. Composition according to claims 8-9, wherein said semi-solid form or liquid form has viscosity between 1 and 2000 mPa·s⁻¹, and pH ranging from 3 to 5.

11. Composition according to claims 5-10, dispensable by pump device (dispenser for semisolid forms, conventional spray, upside down spray), with or without cannula, syringe, catheter, for the on-site application of the preparation.

12. Composition according to claims 5-11 where HANa, VB and GPI are present in the following ranges in weight percentage with respect to their sum: 20-50% HANa, 40-60 %GPI, 1-15% VB.

13. A composition as described in claims 5-12, for topical use in the prevention IBA015WEP or treatment of inflammatory and/or painful conditions of the skin and/or mucosa.

14. Multi-component kit comprising the composition of claim 7 and for reconstitution and topical administration of a composition according to claim 7, in which the verbascoside is packaged separately from the aqueous ingredients.

15. Process for the preparation of a composition according to claims 5-12, comprising associating HANa, VB and GPI with one or more pharmaceutically acceptable excipients.

## Patentansprüche

1. Kombination aus Hyaluronsäure-Natriumsalz (HANa), Verbascosid (VB) und Glycerophosphoinositol (GPI).

2. Kombination nach Anspruch 1 zur Verwendung in der Therapie.

3. Kombination nach den Ansprüchen 1-2 für die topische Verwendung bei der Prävention oder Behandlung von entzündlichen und/oder schmerzhaften Erkrankungen der Haut und/oder der Schleimhaut.

4. Kombination nach den Ansprüchen 1-3 zur Verwendung bei der Prävention oder Behandlung von entzündlichen und/oder schmerzhaften Erkrankungen des weiblichen und männlichen Genitalapparates, insbesondere Vulvodynie, Vestibulitis, Urethritis oder für andere entzündliche Erkrankungen wie Geschwüre, Druckgeschwüre, Venenentzündung, Dermatitis, Akne, Psoriasis, Hämorrhoiden.

5. Stabile pharmazeutische Zusammensetzung, aufweisend die Kombination gemäß Anspruch 1, in der Gegenwart von einem oder mehreren pharmazeutisch annehmbaren Hilfsstoffen.

6. Zusammensetzung nach Anspruch 5, frei von wässrigen Bestandteilen.

7. Zusammensetzung nach Anspruch 5, mit wässrigen Bestandteilen, wobei das Verbascosid getrennt von den wässrigen Bestandteilen verpackt ist.

8. Zusammensetzung nach den Ansprüchen 5-7 in fester, halbfester oder flüssiger Form; als Monodosis oder Mehrfachdosis.

9. Zusammensetzung nach den Ansprüchen 5-7, formuliert als: lyophilisierte Form, halbfeste Zweikomponentenform, mukoadhäsive Lösung oder Pulver.

10. Zusammensetzung nach den Ansprüchen 8-9,
wobei die halbfeste Form oder flüssige Form eine Viskosität zwischen 1 und 2000 mPa·s⁻¹ und einen pH-Wert im Bereich von 3 bis 5 aufweist.

11. Zusammensetzung nach den Ansprüchen 5-10, die sich abgeben lässt durch eine Pumpvorrichtung (Spender für halbfeste Formen, konventionelle Sprühspender, auf den Kopf gestellt Sprühspender), mit oder ohne Kanüle, Spritze, Katheter, für die vor Ort erfolgende Anwendung der Zubereitung.

12. Zusammensetzung nach den Ansprüchen 5-11, wobei HANa, VB und GPI in den folgenden Bereichen in Gewichtsprozent in Bezug auf ihre Summe vorhanden sind: 20-50% HANa, 40-60% GPI, 1-15% VB.

13. Zusammensetzung nach den Ansprüchen 5-12 für die topische Verwendung bei der Prävention oder Behandlung von entzündlichen und/oder schmerzhaften Erkrankungen der Haut und/oder der Schleimhaut.

14. Mehrkomponenten-Kit, aufweisend die Zusammensetzung nach Anspruch 7 und zur Rekonstitution und topischen Anwendung einer Zusammensetzung nach Anspruch 7, wobei das Verbascosid getrennt von den wässrigen Bestandteilen verpackt ist.

15. Verfahren zur Herstellung einer Zusammensetzung nach den Ansprüchen 5-12, das das Kombinieren von HANa, VB und GPI mit einem oder mehreren pharmazeutisch annehmbaren Hilfsstoffen beinhaltet.

## Revendications

1. Association de sel de sodium d'acide hyaluronique (HANa), de verbascoside (VB) et de glycéro-phospho-inositol (GPI).

2. Association selon la revendication 1, pour une utilisation en thérapie.

3. Association selon les revendications 1 à 2 pour une utilisation topique dans la prévention ou le traitement de conditions inflammatoires et/ou douloureuses de la peau et/ou des muqueuses.

4. Association selon les revendications 1 à 3, pour une utilisation dans la prévention ou le traitement de conditions inflammatoires et/ou douloureuses des appareils génitaux féminin et masculin, en particulier la vulvodynie, la vestibulodynie, l'urétrite, ou pour d'autres conditions inflammatoires comme les ulcères, les ulcères de décubitus, la phlébite, la dermatite, l'acné, le psoriasis, les hémorroïdes.

5. Composition pharmaceutique stable comprenant l'association selon la revendication 1, en présence d'un ou de plusieurs excipients pharmaceutiquement acceptables.

6. Composition selon la revendication 5, ne comprenant pas d'ingrédients aqueux.

7. Composition selon la revendication 5, comprenant des ingrédients aqueux, dans laquelle le verbascoside est conditionné séparément desdits ingrédients aqueux.

8. Composition selon les revendications 5 à 7, sous forme solide, semi-solide ou liquide ; unidose ou multidose.

9. Composition selon les revendications 5 à 7, formulée comme suit : forme lyophilisée, forme bi-composant semi-solide, solution muco-adhésive, ou poudre.

10. Composition selon les revendications 8 à 9, dans laquelle ladite forme semi-solide ou liquide présente une viscosité entre 1 et 2000 mPa•s⁻¹, et un pH de 3 à 5.

11. Composition selon les revendications 5 à 10, pouvant être distribuée à l'aide d'un dispositif de pompe (distributeur pour les formes semi-solides, vaporisateur conventionnel, vaporisateur à l'envers), avec ou sans canule, seringue, cathéter, pour l'application sur place de la préparation.

12. Composition selon les revendications 5 à 11, dans laquelle HANa, VB et GPI sont présents dans les plages suivantes en pourcentage de poids par rapport à leur somme : 20 à 50 % de HANa, 40 à 60 % de GPI, 1 à 15 % de VB.

13. Composition telle que décrite selon les revendications 5 à 12 pour une utilisation topique dans la prévention ou le traitement de conditions inflammatoires et/ou douloureuses de la peau et/ou des muqueuses.

14. Kit à plusieurs composants comprenant la composition de la revendication 7 et pour la reconstitution et l'administration topique d'une composition selon la revendication 7, dans lequel le verbascoside est conditionné séparément des ingrédients aqueux.

15. Procédé de préparation d'une composition selon les revendications 5 à 12, comprenant l'association de HANa, VB et GPI avec un ou plusieurs excipients pharmaceutiquement acceptables.
